# EUROPEAN PATENT SPECIFICATION

(11) **EP 2 808 018 B1**
(45) Date of publication and mention of the grant of the patent: **14.03.2018**
(21) Application number: 13740913.2
(22) Date of filing: 25.01.2013
(51) Int. Cl.: A61K 31/426, A61P 3/10

(54) **THERAPEUTIC AGENT FOR DIABETES**
THERAPEUTISCHER WIRKSTOFF GEGEN DIABETES
AGENT THÉRAPEUTIQUE POUR LE DIABÈTE

(30) Priority: 27.01.2012 JP 2012015351
(43) Date of publication of application: 03.12.2014
(73) Proprietor: Teijin Pharma Limited, Tokyo 100-0013 (JP); National University Corporation Tottori University, Tottori-shi Tottori 680-8550 (JP)
(72) Inventor: HISATOME, Ichiro, Yonago-shi Tottori 683-8503 (JP); TSUJIMOTO, Shunsuke, Hino-shi Tokyo 191-0065 (JP); SHIRAKURA, Takashi, Hino-shi Tokyo 191-0065 (JP); HIRAMITSU, Shinya, Nagoya-shi Aichi 457-0047 (JP)
(74) Representative: Carpmaels & Ransford LLP
(86) International application number: PCT/JP2013/051627
(87) International publication number: WO 2013/111870

(56) References cited:
- EP-A1- 0 513 379
- EP-A1- 2 586 442
- WO-A1-92/09279
- WO-A1-2007/018687
- WO-A1-2010/045636
- WO-A1-2011/022757
- WO-A1-2011/162390
- WO-A2-2006/012438
- WO-A2-2007/062028
- JP-A- 2007 210 978
- JP-A- 2009 501 795
- US-A1- 2011 311 620
- LAURA G SÁNCHEZ-LOZADA ET AL: "Effects of febuxostat on metabolic and renal alterations in rats with fructose-induced metabolic syndrome", AJP: RENAL PHYSIOLOGY, AMERICAN PHYSIOLOGICAL SOCIETY, vol. 294, no. 4, 1 April 2008 (2008-04-01) , pages F710-F718, XP002621471, ISSN: 0363-6127, DOI: 10.1152/AJPRENAL.00454.2007 [retrieved on 2008-01-01]
- MACDONALD,P. ET AL.: 'Febuxostat (FEB) vs. Allopurinol (ALLO) in treating the hyperuricemia of gout in diabetic patients' PHARMACOTHERAPY vol. 31, no. 10, 2011, page 381E, XP055157622
- BECKER,M.A. ET AL.: 'Febuxostat (vs. Allopurinol) In Treating the Hyperuricemia of Gout In Diabetic Patients' ARTHRITIS & RHEUMATISM vol. 63, no. 10, 2011, page S399, XP055164395
- CANTU-MEDELLIN,N. ET AL.: 'Manipulation of Xanthine Oxidase-Derived Reactive Species Reduces Obesity-Induced Inflammation and Impairment of Glucose Tolerance' FREE RADICAL BIOLOGY & MEDICINE vol. 53, November 2012, pages S95 - S96, XP055157638
- SONI,J.P. ET AL.: 'Febuxostat: the new generation novel xanthine oxidase inhibitors' INTERNATIONALE PHARMACEUTICA SCIENCIA vol. 1, no. 1, 2011, pages 107 - 115, XP055157640

## Description

### [Technical field]

The present invention relates to a therapeutic agent or prophylactic agent for diseases caused by glucose metabolism disorders comprising, as an active ingredient, a 2-phenylthiazole compound represented by formula (I) or a pharmaceutically acceptable salt thereof. In particular, the invention relates to a compound for use as defined in the claims.

### [Background art]

A glucose metabolism disorder is the condition of producing abnormality in glucose metabolism, and diabetes mellitus is a typical disease caused by glucose metabolism disorders. Diabetes mellitus is classified into type1 and type 2 diabetes mellitus, and type 1 diabetes mellitus is known to have an unequivocal deficiency of insulin actions and type 2 diabetes mellitus is known to have metabolic abnormalities with hyperglycemia induced by an unequivocal or relative insufficiency of insulin actions. That is, type 2 diabetes is a metabolic disorder with the elevation of blood glucose levels, blood glucose levels 2 hours after glucose load, fasting blood sugar (FBS) levels, and hemoglobin A1c (HbA1c) as predominant manifestations and develops characteristic complications over the years.

In recent years, it is also regarded as a problem that not only complications but also type 2 diabetes mellitus, impaired glucose tolerance and insulin resistance similarly caused by glucose metabolism disorders are known to cause high risk for cerebrovascular diseases and coronary artery diseases as well as hypertension, lipid metabolism abnormality, and obesity.

On the other hand, xanthine oxidase inhibitors have been applied in clinical settings as therapeutic agents for gout and hyperuricemia.

It has been reported that allopurinol, which has been used as an xanthine oxidase inhibitor for a long time, has improved insulin resistance associated with hyperuricemia in a model animal (Non-patent literature 1), however, there is no report that blood glucose levels in patients with type 2 diabetes mellitus and in the model animals have been improved. On the contrary, there is a conflicting report that allopurinol was not effective in improving diabetes mellitus, while allopurinol has the ability to lower uric acid levels in mice with hyperuricemia (Non-patent literature 2). Furthermore, non-patent literature reported that allopurinol lowered HbA1c, an index of type 2 diabetes mellitus in human (Non-patent literature 3). However, the dose of allopurinol used in the report was much higher than that normally used in clinical settings. Moreover, several reports indicated that allopurinol at a dose commonly used in clinic settings showed no effect of improving diabetes mellitus among patients with diabetes mellitus (Non-patent literature 4 and 5). Rather, many negative reports have shown, for example, exacerbation in the index of diabetes mellitus by allopurinol (Non-patent literature 6 and 7), improvement of the index of diabetes mellitus in patients treated with allopurinol by discontinuation of medication (Non-patent literature 7 and 8), and the like.

Moreover, 1-(3-cyano-4-neopentyloxyphenyl) pyrazole-4-carboxylic acid as another xanthine oxidase inhibitor has been reported to improve insulin resistance and impaired glucose tolerance, besides lowering the uric acid levels, but with no effect on FBS (Patent literature 1).

It has been known that 2-phenylthiazole compounds such as 2-(3-cyano-4-isobutyloxyphenyl)-4-methyl-5-thiazole carboxylic acid (generic name: febuxostat) and the like used in the present invention show the reduction of uric acid levels resulted from their potent inhibitory activities of xanthine oxidase, thus they are useful as therapeutic agents for hyperuricemia and gout (Non-patent literature 9). Moreover, it has been apparent that 2-phenylthiazole compounds are potential therapeutic agents for kidney disease (Patent literature 2), hypertension (Patent literature 3), and the like other than hyperuricemia and gout. However, the possibility that 2-phenylthiazole compounds are potential therapeutic agents for type 2 diabetes mellitus has not been shown.

### [List of citations]

### [Patent literature]

Patent literature 1: Japanese Unexamined Patent Application Publication No. 2007-210978
Patent literature 2: Published Japanese Translation of PCT International Publication for Patent Application No. 2010-509372
Patent literature 3: Published Japanese Translation of PCT International Publication for Patent Application No. 2009-503094

### [Non-patent literature]

Non-patent literature 1: American Journal of Physiology Renal Physiology 2006; 290: F625-F631
Non-patent literature 2: American Journal of Physiology Renal Physiology 2009; 297: F481-488
Non-patent literature 3: Blood Pressure 2011; 20: 182-187
Non-patent literature 4: Biomedicine and Pharmacotherapy 2004; 58: 546-550
Non-patent literature 5: Iranian Journal of Kidney Diseases 2010; 4(2): 128-132
Non-patent literature 6: Journal of The Medical Association of Thailand 1991; 74: 80-86
Non-patent literature 7: Diabetes Care 1998; 21(1): 192-193
Non-patent literature 8: The British Journal of Diabetes & Vascular Disease 2004; 4: 422
Non-patent literature 9: Arthritis and Rheumatism 2008; 59: 1540-1548

### [Disclosure of the invention]

### [Problem to be solved by the invention]

The purpose of the present invention is to provide a novel therapeutic or prophylactic agent for diseases caused by glucose metabolism disorders, in particular diabetes mellitus, which is not accompanied by hyperuricemia.

### [Means to solve the problem]

As a result of strenuous efforts and investigations in order to solve the above-mentioned problem, the present inventors have found that febuxostat or a pharmaceutically acceptable salt thereof possesses an effect of treating or preventing diseases caused by glucose metabolism disorders.
pharmaceutically acceptable salt thereof.

### [Advantageous effects of invention]

According to the present invention, diabetes mellitus which is not accompanied by hyperuricemia can be treated or prevented by using febuxostat or a pharmaceutically acceptable salt thereof.

### [Description of embodiments]

Febuxostat, which is 2-(3-cyano-4-isobutyloxyphenyl)-4-methyl-5-thiazole carboxylic acid can be produced by the heretofore known methods such as the method disclosed in WO92/09279 and the like.

Diabetes mellitus in the present invention is defined as a disease group associated with various characteristic metabolic disorders, wherein chronic hyperglycemia resulted from insufficiency of insulin actions is a predominant manifestation.

In the present invention, type 2 diabetes mellitus is defined as either one of the following conditions, wherein FBS levels are greater than or equal to 126 mg/dL, glucose levels at 2 hours after a 75-g glucose tolerance test are greater than or equal to 200mg/dL, random blood glucose levels are equal to or greater than 200 mg/dL, or HbA1c is equal to or greater than 6.5% (NGSP level. In case of JDS level, 6.1% or more).

In the present disclosure impaired glucose tolerance is defined as a condition either when FBS levels are equal to or greater than 110 mg/dL and below 126 mg/dL, or glucose levels at 2 hours after a 75-g glucose tolerance test are equal to or greater than 140 mg/dL and below 200 mg/dL.

Insulin resistance in the present disclosure is defined as a condition, wherein the tissue response to insulin decreases and insulin actions are exhibited less.

The compound febuxostat can be converted to pharmaceutically acceptable salts thereof if needed. Such examples include salts with inorganic acids such as hydrochloric acid, hydrobromic acid, hydriodic acid, sulfuric acid, nitric acid, phosphoric acid, carbonic acid and the like; salts with organic acids such as formic acid, acetic acid, propionic acid, trifluoroacetic acid, phthalic acid, oxalic acid, malonic acid, succinic acid, fumaric acid, maleic acid, lactic acid, malic acid, tartaric acid, citric acid, benzoic acid, methanesulphonic acid, ethanesulfonic acid, benzenesulfonic acid, *p*-toluenesulfonic acid, and the like; salts with amino acids such as lysine, arginine, ornithine, glutamic acid, aspartic acid, and the like; salts with alkali metals such as sodium, potassium, lithium, and the like; salts with alkaline earth metals such as calcium, magnesium, and the like; salts with metals such as aluminium, zinc, iron, and the like; salts with organic bases such as methylamine, ethylamine, *t*-octylamine, diethylamine, trimethylamine, triethylamine, ethylenediamine, piperidine, piperazine, pyridine, picoline, ethanolamine, diethanolamine, triethanolamine, cyclohexylamine, dicyclohexylamine, N-methylglucamine, tris(hydroxymethyl)aminomethane, N,N'-dibenzylethylenediamine, and the like; ammonium salts; and the like.

An active ingredient in the present invention can be used in any one of several dosage forms including solid formulation, semisolid formulation, liquid formulation and the like, and used in any one of applicable preparations including oral and parenteral preparations (injection, transdermal formulation, ophthalmic solution, suppositories, transnasal formulation, inhalant, and the like).

The therapeutic or prophylactic agent of the present invention for diabetes mellitus comprising, as an active ingredient, the 2-phenylthiazole compound or a pharmaceutically acceptable salt thereof is prepared by using carriers, diluting agents, and other additives which are routinely used for formulation process. The carriers and diluting agents for formulation may be either solid or liquid. Examples include lactose, magnesium stearate, starch, talc, gelatin, agar, pectin, gum arabic, olive oil, sesame oil, cocoa butter, ethylene glycol, and the like, and other materials used routinely for formulation. Administration may be in any oral preparations such as tablets, pills, capsules, granules, powders, liquid solutions and the like, or any forms of injection such as intravenous, intramuscular, or in any forms of parenteral administrations such as suppositories, transdermal preparations and the like.

The dosage of the active ingredient in the present invention means a therapeutically effective dosage to treat or prevent diabetes mellitus, and can be determined by the symptoms, age, body weight of patients, varieties of combination therapy, frequencies of treatment, desired advantageous effects, the route of administration, or the like. Administration may be daily or intermittently. The frequency of administration is generally 1 to 3 times/day, generally approximately 0.5 to 1000 mg/time per adult (preferably 10 to 120 mg) and 0.5 to 3000 mg/day (preferably 10 to 360 mg, more preferably 10 to 120 mg). Furthermore, the frequency of administration may be 1 to 3 times/week; administration is generally approximately 0.5 to 1000 mg/time per adult. The formulation is preferably prepared to fulfill such conditions.

### [Examples]

### [Example 1] Study of effect on insulin resistance, impaired glucose tolerance, random blood glucose level in mice loaded with high-fat diet.

Febuxostat (2-(3-cyano-4-isobutyloxyphenyl)-4-methyl-5-thiazole carboxylic acid) was administered to mice loaded with a high-fat diet and compared to a control group (Vehicle group) in order to study the effect of febuxostat on insulin resistance, impaired glucose tolerance, and random blood glucose levels. Furthermore, blood uric acid levels were measured to study the relationship of febuxostat with blood uric acid levels.

### [Methods]

Male C57BL/6J mice (age of 8 weeks) were loaded with a high-fat diet, with the proportion of fat-derived calories over total calories (Fat Kcal%) of 60%. Herewith, insulin resistance, impaired glucose tolerance, and diabetes mellitus are developed. In a group of febuxostat treatment (Febuxostat group), febuxostat was dissolved in tap water at a dose of 3 mg/kg/day and was administered to the mice as daily drinking water at the same time of the initiation of loading with a high-fat diet. Mice in the Vehicle group were given tap water. Moreover, in the normal animal group, mice were reared with a regular diet and tap water in the same period as the Febuxostat group and the Vehicle group.

Ten weeks after the start of the high-fat diet and the administration of febuxostat, an insulin tolerance test was carried out to assess random blood glucose levels and insulin resistance. More specifically, 1.5 U/kg of insulin was intraperitoneally administered to the mice and blood glucose levels were measured at 0 minute, 30 minutes, 60 minutes, 90 minutes, 120 minutes after the administration of insulin. Moreover, 12 weeks after the initiation of the high-fat diet and the administration of febuxostat, 0.5 g/kg of glucose was intraperitoneally administered to the mice starved for 4 hours to assess 2-hour blood glucose levels after glucose load. Blood glucose levels were measured at 120 minutes after the administration of glucose. Moreover, on the last day of the administration, blood sampling was carried out to measure blood uric acid levels.

### [Results]

### (i) Random blood glucose level

Results of the measurement of random blood glucose levels are shown in Table 1. Elevation of random blood glucose levels was observed in the Vehicle group in comparison to the normal animal group. On the other hand, lowering random blood glucose levels in the Febuxostat group was observed. Therefore, it was suggested that febuxostat had an effect of improving diabetes mellitus.

### [Table 1]

**Table 1**

| Group | Normal animal | Animal with disease | |
|---|---|---|---|
| | | Vehicle | Febuxostat |
| Random blood glucose level (mg/dL) | 155±9 | 270±9 | 244±7* |

| | | | |
|---|---|---|---|
| The value indicates mean value ± standard deviation (each group; n=9 to 10) * : P<0.05 vs vehicle (Unpaired Student's *t*-test) | | | |

### (ii) Insulin tolerance test

The changes in the blood glucose levels after the loading of insulin are shown in Table 2. In comparison to the normal animal group, a reduced lowering of blood glucose levels by the administration of insulin and an increase of blood glucose level AUC (0-2 hour) after the insulin administration were observed in the Vehicle group and therefore, the development of insulin resistance in the Vehicle group was confirmed. On the other hand, in comparison to the Vehicle group, greater lowering of blood glucose levels by the administration of insulin and a reduction of blood glucose level AUC (0-2hour) were observed in the Febuxostat group. Therefore, it was suggested that febuxostat had an effect of improving insulin resistance.

### [Table 2]

**Table 2**

| Group | | Normal animal | Animal with disease | |
|---|---|---|---|---|
| | | | Vehicle | Febuxostat |
| Ratio of blood glucose levels relative to the value before insulin administration (%) | 30 min | 55±4 | 75±6 | 66±4 |
| | 60 min | 37±4 | 73±6 | 62±3 |
| | 90 min | 45±4 | 82±8 | 71±4 |
| | 120 min | 51±3 | 100±8 | 91±5 |
| Blood glucose level AUC (0-2 hour) (mg·hr/dL) | | 156±7 | 449±19 | 371±17* |

| | | | | |
|---|---|---|---|---|
| The value indicates mean value ± standard deviation (each group; n=9 to 10) * : P<0.05 vs vehicle (Unpaired Student's *t*-test) | | | | |

### (iii) Blood glucose levels 2 hours after the glucose load

Results of the measurement of blood glucose levels 2 hours after the loading of glucose are shown in Table 3. In comparison to the normal animal group, blood glucose levels 2 hours after the loading of glucose in the Vehicle group were elevated. However, the elevation of blood glucose levels 2 hours after the loading of glucose in the Febuxostat group was suppressed compared to the Vehicle group. Therefore, it was suggested that febuxostat had an effect of improving impaired glucose tolerance and diabetes mellitus.

### [Table 3]

**Table 3**

| Group | Normal animal | Animal with disease | |
|---|---|---|---|
| | | Vehicle | Febuxostat |
| Blood glucose levels 2 hours after glucose load (mg/dL) | 185±12 | 468±18 | 396±18* |

| | | | |
|---|---|---|---|
| The value indicates mean value ± standard deviation (each group; n=9 to 10) * : *P*<0.05 vs vehicle (Unpaired Student's *t*-test) | | | |

### (iv) Blood uric acid levels

Results of the measurement of the blood uric acid levels are shown in Table 4. There was no difference in uric acid levels between the normal animal and the Vehicle groups. On the contrary, a decline in the blood uric acid levels was observed in the Febuxostat group.

### [Table 4]

**Table 4**

| Group | Normal animal | Animal with disease | |
|---|---|---|---|
| | | Vehicle | Febuxostat |
| Blood uric acid levels (mg/dL) | 1.0±0.1 | 1.0±0.1 | 0.7±0.1 |

| | | | |
|---|---|---|---|
| The value indicates mean value ± standard deviation (each group; n=9 to 10) | | | |

From those results, it was suggested that febuxostat possessed an effect of improving insulin resistance, impaired glucose tolerance, and diabetes mellitus not only in individuals with hyperuricemia but also in those showing normal blood uric acid levels.

### [Example 2] Study of the effect on impaired glucose tolerance in mice loaded with high-fat diet.

Febuxostat was administered to disease mice loaded with a high-fat diet and the results were compared to a Vehicle group in order to study the effect of febuxostat on the impaired glucose tolerance.

### [Methods]

Male C57BL/6J mice (8 week-old) were loaded with a high-fat diet with the proportion of fat-derived calories over the total calories (Fat Kcal%) of 60%. Herewith, insulin resistance, impaired glucose tolerance, and diabetes mellitus are developed. At the same time of the initiation of loading high-fat diet, febuxostat dissolved in tap water at 3 mg/kg/day was administered to mice as drinking water in the Febuxostat group and mice in the Vehicle group were reared by administration of tap water as drinking water.

Twelve weeks after the loading of a high-fat diet and the administration of febuxostat, a glucose tolerance test was carried out to assess impaired glucose tolerance. More specifically, the mice were made to fast for 4 hours; thereafter 0.5 g/kg of glucose was intraperitoneally administered to the mice. And blood sampling was conducted at 0 minute, 15 minutes, 30 minutes, 60 minutes, 90 minutes, and 120 minutes after glucose load respectively to measure glucose levels.

### [Results]

The results of blood glucose levels during the study are shown in Table 5. Since the elevation of blood glucose levels in the Febuxostat group was reduced in comparison to the Vehicle group, it was suggested that febuxostat possessed an effect of improving impaired glucose tolerance.

### [Table 5]

**Table 5**

| Group | | Animal with disease | |
|---|---|---|---|
| | | Vehicle | Febuxostat |
| Blood glucose levels (mg/dL) | 0 min | 219±11 | 214±17 |
| | 15 min | 407±38 | 368±29 |
| | 30 min | 429±29 | 378±26 |
| | 60 min | 417±50 | 349±35 |
| | 90 min | 381±41 | 323±43 |
| | 120 min | 372±20 | 279±31* |

| | | | |
|---|---|---|---|
| Values indicate mean values ± standard deviation (each group; n=5 to 6) * : *P*<0.05 vs vehicle (Unpaired Student's *t*-test) | | | |

### [Example 3] Administration of febuxostat preparation to patients with hyperuricemia

The effect of febuxostat preparation was studied in patients with hyperuricemia having blood uric acid levels of 7.0 mg/dL or more. The study aimed at patients aged 20 or more having equal to or greater than 7.0 mg/dL of blood uric acid levels with diabetes mellitus having had no administration of urate lowering agents. However, patients who are judged not eligible for the test by their physician-in-charge were not subjected for the test, such as those who have an estimated glomerular filtration rate below 30; patients with hypersensitivity to febuxostat preparation in their past medical history; patients whose hepatic function (aspartate aminotransferase, and alanine aminotransferase) is equal to or greater than twice the criterion measure at trial site for the administration; patients who have complication with chronic hepatic disease, malignant tumor, active infectious disease or inflammatory disease and the like.

The febuxostat preparation was administered to said patients at 10 mg once daily for 4 weeks; thereafter febuxostat was administered at the increased dose to 20 mg once daily for 4 weeks, additionally 40 mg of febuxostat preparation was administered to said patients once daily for 4 weeks. Before and 12 weeks after the administration of the febuxostat preparation, blood sampling was carried out to measure HbA1c, FBS and blood uric acid levels. To patients who were treated with allopurinol, the febuxostat preparation in the same schedule described above was administered after the discontinuation of allopurinol.

Results of the measurement of HbA1c, FBS and blood uric acid levels before and 12 after the administration of the febuxostat preparation are shown in Table 6. The febuxostat preparation reduced HbA1c, FBS and blood uric acid levels as compared to those before administration.

From the results described above, it was shown that the febuxostat preparation improved diabetes mellitus within a clinical dosage that is used for the treatment of hyperuricemia.

### [Table 6]

**Table 6**

| | Before administration | | | 12 weeks after administration |
|---|---|---|---|---|
| | N | Mean±standard deviation | N | Mean±standard deviation |
| HbA1c (%) | 11 | 6.75±1.82 | 10 | 6.58±1.90* |
| FBS (mg/dL) | 11 | 140.09±70.51 | 10 | 126.90±66.10 |
| Serum uric acid levels (mg/dL) | 11 | 8.24±0.91 | 11 | 4.43±1.37** |

| | | | | |
|---|---|---|---|---|
| * : *P*<0.05, ** : *P*<0.0001, (paired *t*-test) [Industrial applicability] | | | | |

## Claims

1. A compound for use in treating or preventing diabetes mellitus, which is not accompanied by hyperuricemia, wherein the compound is 2-(3-cyano-4-isobutyloxyphenyl)-4-methyl-5-thiazole carboxylic acid or a pharmaceutically acceptable salt thereof.

2. The compound or pharmaceutically acceptable salt thereof for use according to claim 1, wherein diabetes mellitus is type 2 diabetes mellitus.

## Patentansprüche

1. Verbindung zur Verwendung bei der Behandlung oder Prävention von Diabetes mellitus, der nicht von Hyperurikämie begleitet ist, wobei es sich bei der Verbindung um 2-(3-Cyano-4-isobutyloxyphenyl)-4-methyl-5-thiazolcarbonsäure oder ein pharmazeutisch unbedenkliches Salz davon handelt.

2. Verbindung oder pharmazeutisch unbedenkliches Salz davon zur Verwendung nach Anspruch 1, wobei es sich bei dem Diabetes mellitus um Diabetes mellitus vom Typ 2 handelt.

## Revendications

1. Composé pour utilisation dans le traitement ou la prévention du diabète sucré, qui n'est pas accompagné d'hyperuricémie, le composé étant l'acide 2-(3-cyano-4-isobutyloxyphényl)-4-méthyl-5-thiazolecarboxylique ou sel pharmaceutiquement acceptable de celui-ci.

2. Composé ou sel pharmaceutiquement acceptable de celui-ci pour utilisation selon la revendication 1, le diabète sucré étant le diabète sucré de type 2.
